# EUROPEAN PATENT APPLICATION

(11) **EP 2 559 419 A1**
(43) Date of publication of application: **20.02.2013**
(21) Application number: 10846009.8
(22) Date of filing: 17.02.2010
(51) Int. Cl.: A61K 8/06, A61K 8/67, A61K 8/30, A61K 8/19

(54) **METHOD FOR MANUFACTURING A COSMETIC PRODUCT BY FREEZING**

(71) Applicant: García Otero, María Del Carmen, CP 36391 Nigrán-Pontevedra (ES)
(72) Inventor: García Otero, María Del Carmen, CP 36391 Nigrán-Pontevedra (ES)
(86) International application number: PCT/ES2010/000073
(87) International publication number: WO 2011/101498

(57) **Abstract**

Procedimiento de elaboración de un producto cosmético por el método de congelación y ultra congelación. This method for manufacturing have four phases that they are explained in the following order: Manufacture an O/W or W/O emulsion, (oil into wáter emulsion or wáter into oil emulsion),gel or another cosmetic product or support that serves to act as a substrate , obtain an extract of nutriotional substances with ingredients , filter the extract obtained and mix the emulsion with the extract until gets a homogeneous mixture and freeze inmediately the mixture obtained into a low temperature.

## Description

This invention resolves efficiently that problem with a method for manufacturing a cosmetic product using the freezing and deep-freezing method. This method gets that the vitamins and the rest of nutriotional substances arrives more efficiently into the skin. This method consists on manufacturing a product whose substrate will be a cosmetic emulsion which was made before (a emulsion oil into water, a emulsion water into oil or a combination between both emulsions on diferents phases, gel or another kind of cosmetic product or substrate that serves to support the extract).

After this, the laboratory obtains a liquid or powder, natural or sintectic extract whose it is composed by nutritional and benefitional substances to the skin like seaweeds, fruits, vegetables,wine,milk,vitamins, minerals,proteins, aminoacids and others analogous substances that it is posible to add into these any differents and complementary substances to get a more effective product if a news and laters discoveries compounds will determinate a more effective cosmetic.

When the liquid or powder extract was obtained succesfully this one will be added into the cosmetic emulsion made before what was mentioned a few lines ago. Inmediately mixs the emulsion with the extract and inmediately freezes the product. The product will be on freeze condition until the moment which the cosmetic product will be used.

The method consists of four phases in the following order:
1- Manufacture an O/W or W/O emulsion, (oil into water emulsion or water into oil emulsion),gel or another cosmetic product or support that serves to act as a substrate.This formulation obtains a manufactured product that will reach nutritional substances like vitamins, minerals,aminoacids ,etc. This emulsion will serve to act as a substrate, support or base for the extract.
2- Obtain an extract of nutriotional substances with ingredients like vitamins or minerals, aminoacids, proteins, etc. This extract can be on liquido or powder condition that could be obtain by natural or chemistry way.
3- Filter the extract obtained and mix the emulsion with the extract until gets a homogeneous mixture.
4- Freeze immediately the mixture obtained into a low temperature that allows preserves the nutriotional substances intact. The last consumer should defrost the cosmetic product just before to aply in the skin to absorbs all the nutrients, minerals and vitamins on pure condition without any kind of manipulation.

With this method it obtains a manufactured cosmetic product made with nutriotional substances that almost arrive without degradation and with the most efficency as is posible to the consumer's skin. This method gives a specific solution to the problems relationed with the use of complex formulations to obtain more stable vitamins like A, C, E vitamins, etc than can produces irritation skin and this method reduces the need to realize some kind of antiallergic tests of high economic and temporary cost. Almost it solves the problem of the loss of efficiency that the cosmetics suffers principally from the moment in which they are opened especially in case of the vitamin C.

There is revealed to the opportune effects, that in the object that constitutes the present patent they will be able to introduce all those variations and modifications of details that the circumstances and the practice make necessary, always and when with the alterations the essence is not modified.

## Claims

1. Procedure of obtaining of a cosmetic - dermatological composition for the method of the freezing and characterized because the following stages of procedure are carried out:
1.1 Production of a water emulsion in oil or of oil in water, gel, or another type of prepared cosmetic or support that uses as application of the extract.
1.2 Obtaining of a liquid extract or in powder that contains nourishing substances
1.3 Filtration of the mixture.
1.4 Mixture of the extract vegetable - mineral with the prepared cosmetic one or support chosen up to obtaining a homogeneous mixture.
1.5 Immediate Freezing of the mixture leaked in packings or any other type of support adapted for the ideal conservation of the prepared one. The frozen product will remain until it comes to the final consumer.
